# EUROPEAN PATENT APPLICATION

(11) **EP 1 987 868 A1**
(43) Date of publication of application: **05.11.2008**
(21) Application number: 07730328.7
(22) Date of filing: 16.02.2007
(51) Int. Cl.: B01D 11/02, C07C 39/10, C07C 39/11

(54) **METHOD FOR THE INDUSTRIAL USE OF TYROSOL AND HYDROXYTYROSOL CONTAINED IN THE SOLID BY-PRODUCTS OF INDUSTRIAL OLIVE CRUSHING**

(30) Priority: 17.02.2006 ES 200600480
(71) Applicant: Universidad de Granada, 18071 Granada (ES)
(72) Inventor: GARCIA-GRANADOS LOPEZ DE HIERRO, Andrés, E-18071 Granada (ES); PARRA SANCHEZ, Andrés, E-18071 Granada (ES)
(74) Representative: Pons Arino, Angel
(86) International application number: PCT/ES2007/000087
(87) International publication number: WO 2007/093659

(57) **Abstract**

The invention relates to a method for using the natural biophenols, tyrosol and hydroxytyrosol, contained in the industrial by-products that result from the crushing and processing of olives, whether from three-phase or two-phase presses, and the husks and cakes thereof. The method produces a mixture of the two biophenols with a purity of more than 90% and yields of between 0.1 and 1.5% depending on the product and the processed raw material. The invention essentially comprises the selective extraction and fractionation of the resulting mixtures using solvents

## Description

### STATE OF THE ART

Olive growing has great importance in the temperate countries of almost the entire world. Its main use is olive oil, of which more than one million MT is produced in Spain annually. The classic procedures for olive milling and oil production are called "three-phase", both in continuous and discontinuous form. By these methods, in addition to oil, by-products such as olive water, aqueous fraction of the olive with or without the addition of water, and the pomaces of various types, which are generally extracted for an additional use of oil, are produced. At present, in addition to the three-phase procedures, that called "two-phase" is used wherein, in addition to the oil, a mass is obtained which contains the residue of the pulp and, usually although not always, the olive pit, mixed with the vegetation water, giving rise to a by-product which is known with the name of "alpeorujo". A well-established industrial product is the "orujo" (pomace), a solid material with around 8% humidity which, after being extracted in the olive-pomace extraction plant to product olive-pomace oil gives rise to "orujillo" (olive oil waste) as by-product, usually intended to serve as fuel in suitable boilers and more recently also as fuel in electricity co-generation plants. The large quantity of chemical products that the various industrial by-products contain has attracted the attention of researchers, there existing a large quantity of publications and patents destined for the study, isolation and application thereof. Special attention has been paid to the isolation of 3,4-dihydroxyphenylethanol (hydroxytyrosol), on which there exist more than 60 patents destined to protect very varied production processes. Thus, researchers from the University of Granada already developed in 1992 a "Method of use of vegetation water for the production of acids, phenols, alcohols and derivatives by counter-flow methods" (Spanish patent application: 9298236). A method fundamentally aimed at the use of hydroxytyrosol from vegetation water from the olive produced by the three-phase procedure, although applicable to the aqueous part contained in the alpeorujo. This method, consisting of the concentration of vegetation water, de-oiled from the extract, subsequent extraction with ethyl acetate and separation of the content of this extract by chromatography procedures permitted isolating the biophenols present in this extract (fundamentally tyrosol and hydroxytyrosol) with high purity. After this, this University of Granada developed and patented a method for the production of mannitol from these same by-products (Use of olive branches and leaves and olive peduncles and vegetation water for the production of mannitol and derivatives P9300490) (Method for the production of mannitol and derivatives from alpeorujo from olive processing according to the two-phase method, P9300945). On consolidating the milling procedure to the so-called two-phase method (without added water) new works have arisen aimed at the isolation of hydroxytyrosol: (Agric. Food Chem., 50 (23), 6804 - 6811(2002) Production in Large Quantities of Highly Purified Hydroxytyrosol from Liquid-Solid Waste of Two-Phase Olive Oil Processing or "Alpeorujo" Juan Fernández-Bolaños,* Guillermo Rodríguez, Rocío Rodríguez, Antonio Heredia, Rafael Guillén, and Ana Jiménez, with various products patented: Method of obtaining a hydroxytyrosol-rich composition from vegetation water (Spanish patent P200100346 (PCT/ES02/00058). Methods have also been developed to isolate hydroxytyrosol concentrates from pitted olives, treating the vegetation waters with citric acid and subsequently incubating them, to produce the hydroxytyrosol concentrate without solvents (US patents 6197308 and 6165474).

Hydroxytyrosol concentrates are marketed with the names of Hydrox® (http:/www.creagri.com/hidrox/proprietary.html) and related products "Method of obtaining a hydroxytyrosol-rich composition from vegetation water" (US patent 6416808) (http:/www.patentstorm.us/oatents/6416808.html). In Spain, they are marketed with the name of Hytolive® (http:/hytolive.com) (http:/www.nutraingredients.com/news/news.asp?id=38632) and in Portugal as Olidrox® (http:/cotecportugal.pt/cotec/images/pdf/Olidrox.pdf)

In addition to the method described in the patients with previous pitting, in the other isolation processes, the line of action consists of a hydrothermal treatment and provoking a prior hydrolysis of various natural components that structurally contain tyrosol and hydroxytyrosol, with or without catalysis, performing filtration processes with membranes and subsequently isolating these phenols in a greater or lesser degree of purity by separation with various types of exchange resins. On the other hand, a large quantity of processes have been developed for the isolation of antioxidants from olive leaves (for example, European Patent EP1389465).

The University of Granada has an industrial patent (P9601652, W098/04331, Method for the industrial use of 3beta-hydroxyolean-12-en-28-oic acid (oleanolic) and (2alpha,3beta)-2,3-dihydroxy-olean-12-en-28-oic acid (maslinic acid) contained in the olive milling by-products), which permits industrially producing these two acids, separately and in a high degree of purity, from solid by-products of industrial olive milling, by any of the methods now used (presses, continuous in three-phases and in the so-called two-phase), which constitutes an attainable and inexhaustible source thereof.

In the industrial tuning of this patent, it has been possible to isolate, in an industrially profitable manner, a concentrate of natural biophenols by procedures very different to those disclosed in the aforementioned patents. The method used is efficient and very-low cost, also bearing in mind that it relates to the use of a by-product from the general method used for the production of oleanolic and maslinic acids.

### DESCRIPTION OF THE INVENTION

The pomace from the "in three phase" processing, the alpeorujo, from the system known as "two phase" and, in general, from any waste from the processing of the complete olive or its parts which contain olive waste, with or without subsequent processing to make use of the oil they contain, are dried until reaching a suitable degree of humidity for their extraction with hexane (or another solvent or mixture of solvents). In the usual manner of the olive pomace extraction industries, this extraction is performed with an apolar solvent (preferably hexane) and the solvent is later eliminated to give olive-pomace oil.

The oil thus produced, or diluted with hexane, is left to rest, a white precipitate appearing, which can be separated from this oil by filtration and/or centrifugation producing a semisolid product which, suitably washed with an apolar solvent (preferably hexane) gives oleanolic acid, which can be subjected to bleaching processes if desired.

The "orujos" already extracted as abovementioned, and now called "orujillos" will again be extracted with a more polar solvent, preferably ethyl acetate, even with liquefied gases in "supercritical conditions".

The extract in question will be reduced in volume by elimination of the solvent, taking it, preferably, to dryness. The extract, once dried, will be treated with a more polar solvent than ethyl acetate, preferably methanol, water or their mixtures, or with liquefied gases in "supercritical" conditions, producing a solution and a precipitate, which will be separated from the solution by centrifugation and/or filtration. The resulting solution, which will be taken to dryness, fundamentally contains a mixture of hydroxytyrosol and tyrosol, polyols, sugars and other minority products.

This mixture of products is dissolved in water, treated with a base solution, preferably, sodium bicarbonate, to fix the carboxylic acids which it contains (acetic, lactic, etc.) and then it is extracted with a solvent of medium polarity, preferably ethyl acetate. In this way, it is managed to extract approximately 50% by weight of the waste which had been dissolved in water.

The concentrate from this apolar phase gives rise to a biophenol concentrate which, controlled by NMR techniques, contains between 50 and 90% of hydroxytyrosol, between 10 and 30% of tyrosol and between 3 and 10 of other minority compounds, thus managing to eliminate the large majority of polyols, sugars and natural acids.

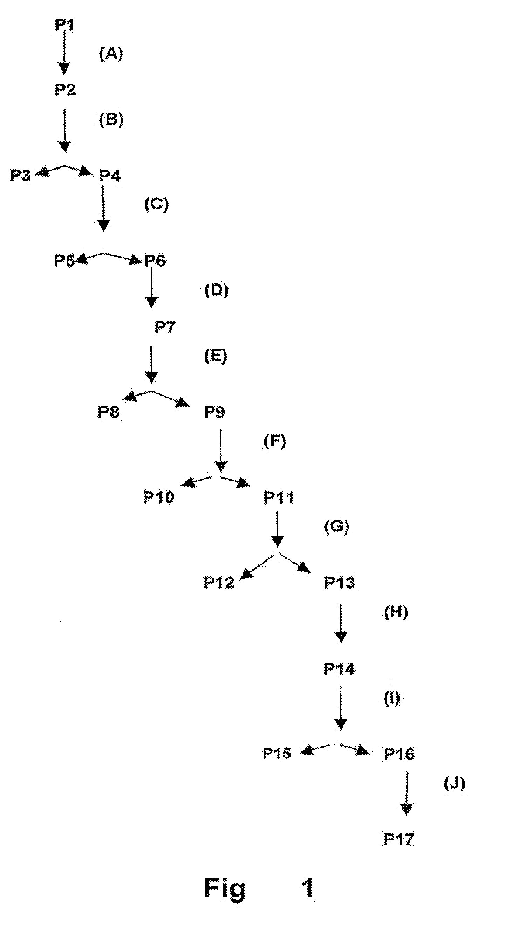

Schematically, the method described is performed via the following operations:
Initially, if necessary, the water the starting product contains (product 1) is eliminated until a water content less than 15% (Pomace, Product 2). Among others, rotary kilns or drying in counter-flow can be used. (Fig. 1, (A)).

Then proceed with the Extraction with hexane of Product 2. It may be in continuous or discontinuous form. (Fig 1, (B)).

Later, it is extracted with ethyl acetate from the solid insoluble in hexane (orujillo, Product 4), from the aforementioned operation or from any commercial source. The extraction can be in continuous or discontinuous form. (Fig. 1, (C))

Product 6 is concentrated totally or partially and it is separated from the precipitate (product 7) by filtration or centrifugation, or by complete concentration producing product 7. (Fig. 1, (D))

Product 7 is washed with apolar solvents, filtering and/or centrifuging the precipitated solid. Thus, a solution is produced which, after eliminating the solvent will give rise to a fatty material (Product 8) and a concentrate (Product 9). (Fig. 1, (E))

Then, the more polar components contained in product 9 are extracted, preferably with alcohols, water or their mixtures, preferably hot. A terpenic concentrate (Product 10) and a polar solution (Product 11) is thus produced. (Fig. 1, (F))

Product 11 is concentrated to dryness to recover, if applicable, the solvent (Product 12) and produce a concentrate (Product 13). (Fig. 1, (G))

The concentrate produced (Product 13) is treated with water, preferably hot, adding a weak base, typically sodium bicarbonate, until it no longer gives off CO₂, to neutralize natural acids present in P9, producing an aqueous solution (Product 14). (Fig. 1, (H))

The repeated extraction of product 14 occurs with a medium polarity solvent, producing an aqueous solution (product 15) and an apolar solution (Product 16). (Fig. 1, (I))

Finally, a complete concentration of the solution produced is performed (Product 16) to produce a solvent-free biophenol concentrate (Product 17). (Fig. 1, (J)) which contains tyrosol and hydroxytyrosol in a concentration over 75%.

### EMBODIMENT OF THE INVENTION

Below, three examples of the practical embodiment of the method object of the present patent are indicated by way of illustrative but non-limitative example:
**Example 1.-** We start from 2500 kg of alpeorujo from olive milling by the method called "two-phase".
   This alpeorujo is dried, in a rotary kiln adapted for this purpose, eliminating the greater part of the water it contains until reaching a humidity of around 8%, thus producing around 1000 kg of a material which can be used, which is then extracted with hexane in facilities typical of the olive-pomace extraction industry. The hexane extract contains around 6% by weight of non-volatile material, and it is largely constituted by the so-called olive-pomace oil (between 5% and 6%), in a highly variable proportion depending on the quality, nature and "history" of the processed pomace. 940 kg of orujillo is also produced which is subjected to extraction with around 1000 litres of ethyl acetate, in the same facilities as those described for extraction with hexane. An ethyl acetate solution is thus produced which is vacuum-concentrated until a content in solid material of around 50% and it is finally taken to dryness by any industrially suitable method, giving around 56 kg of solid material which are treated with 280 litres of hexane, and the non-soluble material, with another 280 litres of boiling methanol, leaving it to cool at rest, then centrifuging. The materials solubilized in methanol are taken to dryness by the methods described for the ethyl acetate extract giving around 23.5 kg of waste which are then treated with 190 litres of boiling water, separating the non-dissolved materials from the aqueous solution by centrifugation. The aqueous solution is treated with hot sodium bicarbonate solution until no CO₂ is given off and it is then extracted successively with three batches of 190 litres of ethyl acetate. The ethyl acetate solutions are combined and they are taken to dryness, producing approximately 6.6 kg of biophenol concentrate, although the quantity and composition of the concentrate depend to a certain extent on the olive variety, the degree of maturity, the "history" of the orujillo, and very particularly on the weight ratio between the materials (pulp and pit) which, as a whole, form the pomace produced by drying.
**Example 2.**- We start from 1000 kg of pomace intended for the production of olive-pomace oil, which is then extracted with hexane in facilities typical of the olive-pomace extraction industry. The hexane extract contains around 6% by weight of non-volatile material, and it is largely constituted by the so-called olive-pomace oil (between 5% and 6%), in a highly variable proportion depending on the quality, nature and "history" of the pomace processed. The orujillo thus produced (approximately 940 kg) is subjected to extraction with ethyl acetate, in the same facilities as those described for extraction with hexane. An ethyl acetate solution is thus produced which is vacuum-concentrated until a content in solid material of around 50% and it is finally taken to dryness by any industrially suitable method, giving around 56 kg of solid material which are treated with 280 litres of hexane, and the non-soluble material, with another 280 litres of boiling methanol, leaving it to cool at rest, then centrifuging. The materials solubilized in methanol are taken to dryness by the methods described for the ethyl acetate extract giving around 23.5 kg of waste which are then treated with 190 litres of boiling water, separating the non-dissolved materials from the aqueous solution by centrifugation. The aqueous solution is treated with hot sodium bicarbonate solution until no CO₂ is given off and it is then extracted successively with three batches of 190 litres of ethyl acetate. The ethyl acetate solutions are combined and they are taken to dryness, producing approximately 6.6 kg of biophenol concentrate, although the quantity and composition of the concentrate depend to a certain extent on the olive variety, the degree of maturity, the "history" of the orujillo, and very particularly on the weight ratio between the materials (pulp and pit) which, as a whole, form the original pomace.
**Example 3.**- We start from 1000 kg of uncleaned orujillo intended for solid fuel, which is subjected to extraction with ethyl acetate, in the same facilities as those described for extraction with hexane. An ethyl acetate solution is thus produced which is vacuum-concentrated until a content in solid material of around 50% and it is finally taken to dryness by any industrially suitable method, giving around 60 kg of solid material which are treated with 300 litres of hexane, and the non-soluble material, with another 300 litres of boiling methanol, leaving it to cool at rest, then centrifuging. The materials solubilized in methanol are taken to dryness by the methods described for the ethyl acetate extract giving around 25 kg of waste which are then treated with 200 litres of boiling water, separating the non-dissolved materials from the aqueous solution by centrifugation. The aqueous solution is treated with hot sodium bicarbonate solution until no CO₂ is given off and it is then extracted successively with three batches of 200 litres of ethyl acetate. The ethyl acetate solutions are combined and they are taken to dryness, producing approximately 7 kg of biophenol concentrate, although the quantity and composition of the concentrate depend to a certain extent on the olive variety, the degree of maturity, the "history" of the orujillo, and very particularly on the weight ratio between the materials (pulp and pit) which, as a whole, form the original orujillo.

### EXPLANATION OF THE FIGURES

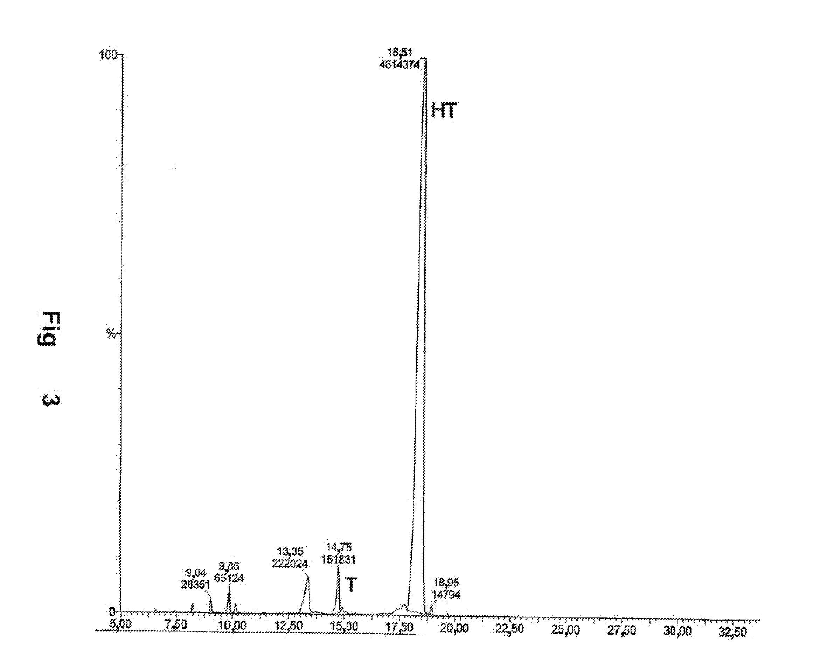

**FIGURE 1****.** It schematically represents the fundamental phases which comprise the extraction process.
   **P1.-** Product 1: Industrial material which results from the milling of olives in any of its procedures, and which contains the waste after the use of olive. For example, alpeorujo.
   **P2.-** Product 2. Pomace.
   **P3.-** Product 3: Olive-pomace oil
   **P4.-** Product 4: Orujillo
   **P5.-** Product 5: Solid disposable waste (mainly skin and olive pit)
   **P6.-** Product 6: Resulting from the extraction from product 4 after its separation from product 5.
   **P7.-** Product 7: Precipitate resulting from the concentration of P8.
   **P8.-** Product 8: Fatty material
   **P9.-** Product 9: Concentrate after washing product 7.
   **P10.-** Product 10: Terpenic concentrate
   **P11.-** Product 11: Polar material solution
   **P12.-** Product 12: Solvent
   **P13.-** Product 13: Product resulting from the elimination of the solvent (P12) from P11.
   **P14.-** Aqueous solution
   **P15.-** Aqueous solution
   **P16.-** Apolar solution
   **P17.-** Solvent-free biophenol concentrate
   **(A).-** (Operation A). Where applicable, elimination of water contained in product 1 until a water content under 15% (Pomace, Product 2). Among others, rotary kilns or drying in counter-flow can be used.
   **(B).-** (Operation B) Where applicable, extraction with hexane of Product 2. It may be in continuous or discontinuous form.
   **(C).-** (Operation C) Extraction with ethyl acetate of the solid insoluble in hexane (orujillo, Product 4), from the aforementioned operation or from any commercial source. The extraction can be in continuous or discontinuous form
   **(D).-** (Operation D) Total or partial concentration of P6 and separation of the precipitate P7 by filtration or centrifugation, or by complete concentration producing P7.
   **(E).-** (Operation E): Washing product 7 with apolar solvents, filtering and/or centrifuging the precipitated solid. Thus, a dilution shall be produced which, after eliminating the solvent will give rise to a fatty material (P8) and a concentrate (P9).
   **(F).-** (Operation F): Extraction of the more polar components contained in product 9, preferably with alcohols, water or their mixtures, preferably hot. A terpenic concentrate (P10) and a polar solution (P11) is thus produced.
   **(G).-** Operation G): Concentration of P11 until dryness to recover, if applicable, the solvent (P12) and produce a concentrate (P3).
   **(H).-** (Operation H): Treatment of the concentrate P13 with water, preferably hot, adding a weak base, typically sodium bicarbonate, until it no longer gives off CO₂, to neutralize natural acids present in P9, producing an aqueous solution (P14).
   **(I).-** (Operation I): Repeated extraction of P14 with a medium polarity solvent, producing an aqueous solution (P15) and an apolar solution (P16).
   **(J).-** (Operation J): Complete concentration of solution 16 to produce a solvent free biophenol concentrate.

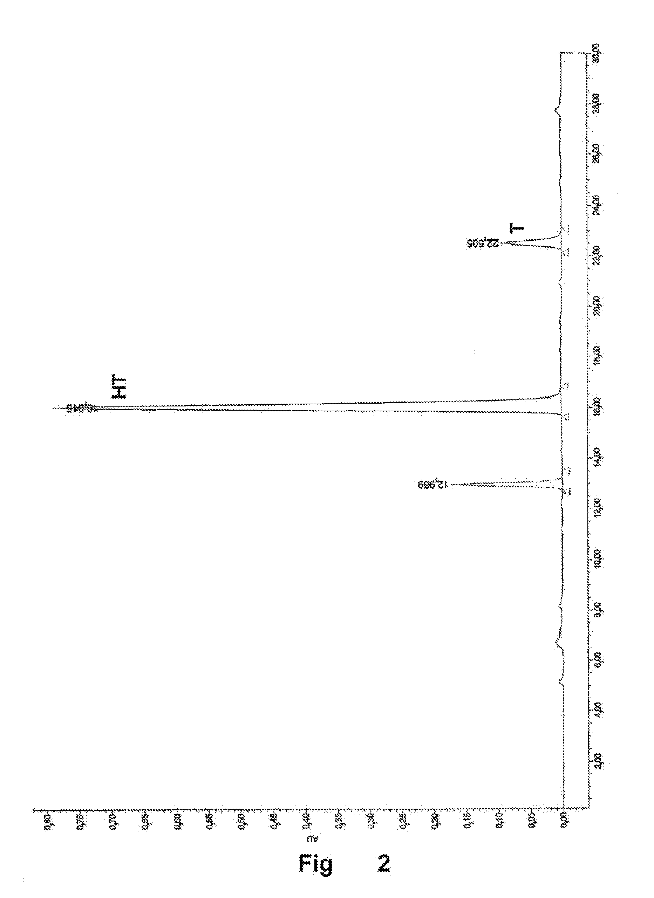

**FIGURE 2****.-**Graphic of a High Performance Liquid Chromatography (HPLC) of a sample obtained according to the method described. The y-axis, AU, indicates Absorbance, the x-axis indicates time in minutes HT indicates Hydroxytyrosol and T indicates Tyrosol.
   **HPLC details:**
   Column: Water Novapack C-18, 4.6 x 250 mm
   Eluent: Water (99.5%); 0.5% Trifluoroacetic
   Flow: 1 ml/minute
   UV detector at 280 nm
**FIGURE 3****.-**Gas Chromatography graphic- Mass spectrometry of a sample obtained according to the described method. The y-axis indicates the relative height, the x-axis the time in minutes, HT indicates Hydroxytyrosol and T indicates Tyrosol.
   **GC-MS details:**
   Sample silanized with BSTFA.
   Column: Capillary HP5MS, 30 m, 0.25 mmID, 0.25 micron film
   Carrier: He 1 ml/min (psi 14)
   Split: without split
   Temperature: 75 to 200°C at 7°C/min; 200 to 300°C at 12°C/min.
   Total ion detector.

## Claims

1. Method for the industrial use of tyrosol and hydroxytyrosol, **characterized in that** their extraction is performed from industrial solid by-products of olive milling with an output of tyrosol and hydroxytyrosol over 90%.

2. Method for the industrial use of tyrosol and hydroxytyrosol, **characterized in that** their extraction is exclusively performed with solvents from industrial solid by-products of olive milling with an output of tyrosol and hydroxytyrosol over 90%.

3. Method for the industrial use of tyrosol and hydroxytyrosol, **characterized in that** their extraction is exclusively performed with solvents from industrial solid by-products of olive milling with an output of tyrosol and hydroxytyrosol over 90% and which comprises the following phases:
a. Elimination of the water the starting product contains until a water content lower than 15%.
b. Extraction with apolar solvent or solvents or their mixtures of the waste produced in a) to produce an apolar solution and a residue insoluble in apolar solvents.
c. Extraction with medium polarity solvent or solvents or their mixtures from the insoluble residue described in b), producing a residue also insoluble in these medium polarity solvents and a solution containing the products of medium polarity.
d. Total or partial concentration of the medium polarity solution produced in c) and separation of the precipitate which is produced by filtration and/or centrifugation, or complete concentration.
e. Washing of the semi-solid material produced in d) with apolar solvents, filtering and/or centrifuging the precipitated solid, producing a solution and an insoluble residue.
f. Extraction of the insoluble residue produced in e), preferably with alcohols, water or their mixtures, preferably hot. A solid residue which is insoluble in these polar solvents and a solution of the material thus solubilized is thus produced.
g. Concentration of the polar solution produced in f) until dryness to recover, where applicable, the solvent used in f) and production of a concentrate.
h. Treatment of the concentrate produced in f) with water, preferably hot, adding a weak base to neutralize natural acids present there, producing a neutralized polar solution.
i. Repeated extraction of the neutralized polar solution with a medium polarity solvent, producing a polar phase and an apolar phase.
j. Complete concentration of the apolar phase to produce a concentrate which contains tyrosol and hydroxytyrosol.

4. Method according to any of the preceding claims, **characterized in that** the starting product is olive milling waste.

5. Method according to any of claims 1 to 3, **characterized in that** the starting product is pomace from olive oil extraction in the modality known as the "three-phase" system or presses.

6. Method according to any of claims 1 to 3, **characterized in that** the starting product is alpeorujo from olive oil extraction in the modality known as the "two-phase" system.

7. Method according to any of claims 1 to 3, **characterized in that** the starting product is orujillos from the olive-pomace extraction plants.
